# EUROPEAN PATENT APPLICATION

(11) **EP 4 082 529 A1**
(43) Date of publication of application: **02.11.2022**
(21) Application number: 22170636.9
(22) Date of filing: 28.04.2022
(51) Int. Cl.: A61K 9/06, A61K 47/36

(54) **A POLYSACCHARIDE SYNERGIC COMBINATION FOR THE INTEGRITY OF MUCOUS MEMBRANES**

(30) Priority: 29.04.2021 IT 202100010916
(71) Applicant: Unifarco S.p.A., 32035 Santa Giustina (BL) (IT)
(72) Inventor: Baratto, Giovanni, 32035 Santa Giustina BL (IT); Busata, Laura, 32035 Santa Giustina BL (IT); Francescato, Stefano, 32035 Santa Giustina BL (IT); Semenzato, Alessandra, 35131 Padova PD (IT); Costantini, Alessia, 35131 Padova PD (IT); Tafuro, Giovanni, 35131 Padova PD (IT)
(74) Representative: Perani & Partners S.p.A.

(57) **Abstract**

Mucosal composition containing a mixture comprising at least three polysaccharides belonging to at least two of the following classes 1, 2, 3 and 4, as classified in Table 1 below

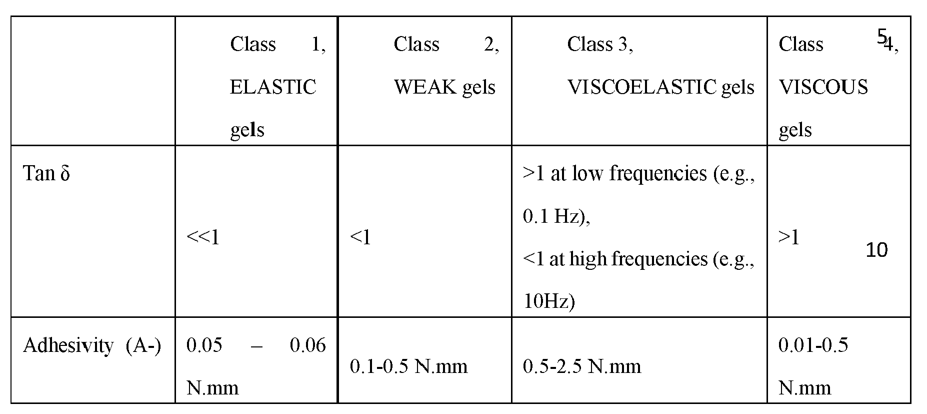

in combination with suitable excipients and/or diluents,
in which said mixture has:
a) at 1Hz: complex module G^{∗} comprised between 1 and 500 Pa and Tan δ comprised between 0.3 and 1
b) Adhesivity A- 0.08 - 5 N.mm and
c) the relative polysaccharides are selected from Table 2 below

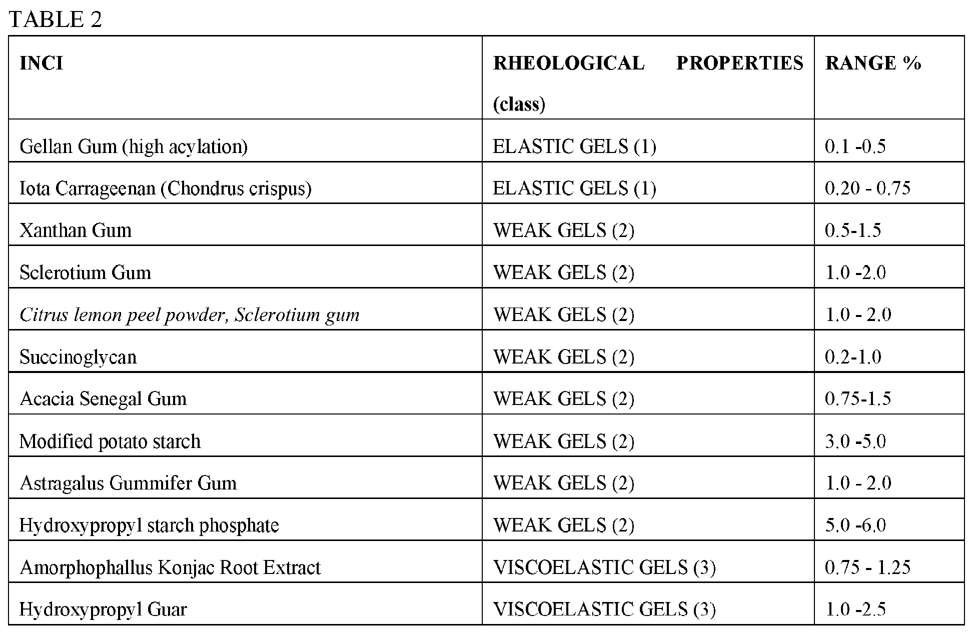

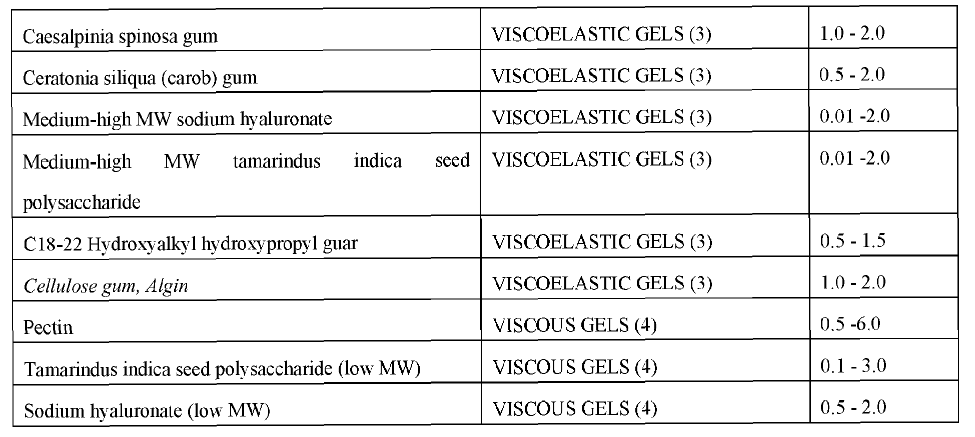

This composition is ideal for application on mucous membranes, particularly oral or nasal, in terms of adhesion, ease of application and compliance and is particularly effective in preventing and treating viral and bacterial infections.

## Description

### FIELD OF THE INVENTION

The present invention relates to compositions containing polysaccharides suitably combined to obtain gels whose technical, application and functional properties are ideal for application on oral or nasal mucous membranes, particularly in terms of adhesion, ease of application and compliance.

### BACKGROUND ART

Polysaccharides are a heterogeneous class of natural polymers, classified according to the primary structure and source from which they are extracted (vegetable, marine, microbial or animal), which have been exploited for centuries due to their availability, low production costs and structural diversity.

Polysaccharides have known biological functions: they protect the body from adverse environmental conditions, in situations of nutrient and water deficiency, in unfavourable temperature conditions and in the presence of high saline concentrations. Polysaccharides can have a structural, energy reserve, protective function, with a barrier function against the entry of foreign organisms and prevention against water loss.

Polysaccharides have always been used as cosmetic active ingredients, because they are easily available from common natural sources such as roots and seeds, while today they also play a fundamental role in formulation technology thanks to their multifunctionality. (J. V. Gruber, 1999). The growing attention to sustainability issues has led to a rapid expansion in the production and use of these raw materials in the industrial, food, cosmetic and pharmaceutical sectors. Furthermore, since they are natural organic polymers obtained by biosynthesis from renewable sources, they have peculiar features such as biocompatibility, biodegradability, bioadhesivity and absence of toxicity, particularly advantageous for use in the cosmetic field (B. Jayawardena et al, 2017).

Most polysaccharides of cosmetic interest are chains consisting of monosaccharides with 6 carbon atoms, such as aldohexoses and uronic acids, which are derived from the oxidation of hydroxyl at position 6 of aldoses (A. Malpede et al., 2007).

The monosaccharide units are linked by glycosidic bonds, each of which is characterized by its own degree of rotation dependent on steric factors and intramolecular interactions.

Anionic, cationic, non-ionic or amphoteric polysaccharides can be distinguished: the anionic charge is typical of many natural polysaccharides, while the cationic charge is diffuse among synthetic seeds, mainly being the result of chemical modifications. The charge contribution is not only decisive for the different sensitivity of the polysaccharide to the presence of electrolytes, but also influences the behaviour in solution and the intra and inter-chain interactions.

In solution they can exist as disordered chains or be organized in helical structures, with variable features depending on the primary structure of the polymer, the nature of the substituents, the intra-chain interactions and the degree of ramifications: in fact, right-handed or left-handed helices are formed, super wound or relaxed, single and aggregated.

The application interest for polysaccharides as rheological modifiers derives mainly from their ability to form gels. The study of the gelling mechanisms is fundamental for understanding the contribution made by the polymer to the physico-chemical features of the formulation.

By definition, gels are semi-solid formulations consisting of a three-dimensional lattice of molecules held together by hydrophobic junction zones. The meshes of this polymeric network give the gel cohesive features typical of elastic solids, and allow to attract and retain molecules of the vehicle. In contrast, the liquid phase is responsible for the viscous and diffusion properties and controls the gel density.

Polysaccharide gels are defined as physical (or type II gels), systems held together by weaker, noncovalent interactions such as hydrogen bonds, ionic interactions, van Der Waals forces, which are established between linear or poorly branched chains. The rupture of these interactions may be induced by simple heating, resulting in the destruction of the three-dimensional network. In fact, physical gels do not maintain their structural integrity.

The chemical-physical properties of the resulting system depend on the molecular weight, the chemical nature of the repeating units, the presence of charged substituent groups, the different ratio of the sugar residues.

The actual degree of swelling, i.e., the amount of water which each polymeric network can absorb, not only depends on the density of hydrophilic groups, but also on the gelling conditions, such as temperature, pH, the presence of other solutes and/or co-solvents and overall ionic strength.

The combined use of polysaccharides is known in the literature of cosmetic formulations for dermal, non-mucosal application. In particular, the applicant refers to the following as the prior art documents
- US 2021/093539 A1: discloses (example 94 on page 20) a composition for topical use with anti-ageing effect and comprising, among other ingredients, a) cellulose gum, b) ceratonia siliqua, c) sclerotium gum, d) Xanthan gum. US 2011/278500 A1: discloses (example 48 in table VII on pages 11-12) an adsorbent composition comprising a) konjac gum as a viscoelastic gel. b) Xanthan gum as a weak gel and c) pectin as a viscous gel.
- CN 106727252 A: discloses in the claims a liquid essence comprising among other ingredients: a) 0.05-0.08% by weight gellan gum (elastic gel), b) 0.1-0.2% by weight Xanthan gum (weak gel) and c) 0.1-0.2% by weight sodium hyaluronate and 0.2-0.3% by weight hydrolysed hyaluronic acid (viscous or viscoelastic gel).
- WO 2010/132857 A1: discloses (in example 3 on page 17) a hydrogel comprising a) konjac gum (viscoelastic gel), b) xanthan gum (weak gel), and c) i-carrageenan (elastic gel);
- US 10398637 B2: discloses a hair formulation (column 4, row 61; -column 5, line 19; column 10, rows 9-21; in formula 4 in example 1 in column 18-19) comprising carrageenan, sclerotium gum, hydroxypropyl guar. Carrageenan is selected from lambda, kappa or iota carrageenan or a mixture of the three.

In a gel for cosmetic use with a predominantly polysaccharide composition, the polysaccharides play a major role not only for functionality but also in imparting texture and sensory features to the product. Each cosmetic product is characterized by a specific texture and each texture, if rationally chosen, can be a vehicle of functional effectiveness: a cosmetic product with adequate texture features to support the requirements of functional effectiveness and capable of meeting the expectations of the consumer will have the initial requirements to retain it.

The concept of texture of cosmetic products is based on what is defined in ISO-5492 (2008), where this term represents the "combination of mechanical, geometric and surface attributes of a product, which are perceived through mechanical, tactile, visual and auditory receptors". It mainly refers to the consistency of the cosmetic product, its smoothness and the ease of withdrawal from the container and application.

In this context, therefore, all the aspects of the product must be rationally evaluated: shape, consistency, rate of absorption, tactile sensibility, visual impact, colour, brightness and smell. As much as sensoriality is a subjective experience, we try to study it as objectively as possible. The global characterization requires the use of different evaluation techniques, combining classic analytical methods and sensory analysis techniques, which allow to analyse, quantify and interpret the sensations evoked by the interaction between the consumer and the product.

The most used analyses include:
- Rheological analysis: instrumental method which allows to study the flow properties of materials. Rheology is considered a valid tool for evaluating the sensory and functional features of the product, especially in the formulation development phase (S.Wang, M.S Kislalioglu, Breuer; 1999). As the sensory properties are closely related to the microscopic structure of the sample, the rheological analyses are functional in the preliminary studies of the formulation, when it is essential to have information regarding the behaviour and properties of the individual raw materials.
- Texture analysis: an instrumental technique that allows to evaluate the texture of a product based on multiple features:
   ∘ Mechanical features such as hardness, texture, adhesivity, cohesiveness and elasticity. Their analysis makes it possible to predict the behaviour of a product during the withdrawal phase from the container and during application.
   ∘ Geometric features, which refer to the internal structure of the product and influence its visual appearance.

The formulation of products suitable for application on buccal mucous membranes, gold and pharyngeal nose requires the formulator to study products with specific texture and application features suited to the application site, in particular in terms of adhesion, ease of application and compliance.

As reported in the Italian Official Pharmacopoeia (FUE XII ed.), oromucosal preparations are solid, semi-solid or liquid preparations containing one or more active substances, intended for administration to the oral cavity and/or throat to obtain a local or systemic effect.

However, the oral cavity has an unfavourable environment for the application of pharmaceutical formulations not only for the complexity of the anatomical structures but also for the constant presence of salivary secretions, mainly of the mucous type.

The mucosa of the oral cavity consists of a multi-layered squamous epithelium supported by a basal membrane, a dense connective lamina propria and a submucosa.

Because of its compact structure and the constant turnover of the cells, the epithelium essentially performs the function of a barrier. Therefore, a damaged epithelium no longer performs this function in an adequate manner.

Consequently, a product for the oral mucosa must have high adhesion features to adhere to the application area, resisting the flushing of saliva and have viscosity features suitable for forming a pleasant and well tolerated protective film.

In FUE, the nasal preparations *(nasalia)* are liquid, semi-solid or solid preparations to be administered into the nasal cavities to achieve a systemic or local effect.

For the purpose of the present invention, certain categories can be distinguished: Nasal drops, nasal powders, semi-solid nasal preparations.

Semi-solid nasal preparations, such as for inhalation preparations, are often administered in the form of sprays. Such formulations require the use of a suitable administration device.

The physical-mechanical properties of pure polysaccharides are known and reported in the literature; however, knowing the rheological and texture properties of individual raw materials allows to overcome the "trial and error" approach and set a rational formulation design based on the specific target product you want to achieve.

In addition, binary or multiple polysaccharide mixtures have unique properties, different from those of the individual materials, and difficult to predict.

### SUMMARY OF THE INVENTION

The applicant has studied several classes of polysaccharides according to rheological behaviour and textures (e.g., weak gels, elastic gels, viscoelastic gels, viscous gels) and has found that the association of these involves a modulation of the viscoelastic and adhesive properties, which makes them particularly suitable for applications on the mucous membranes, in particular nasal and oral.

The object of the present invention are therefore mucosal compositions containing a mixture comprising at least three polysaccharides belonging to at least two of the following classes 1, 2, 3 and 4, as classified in Table 1 below.

**TABLE-1**

| | Class 1, gel ELASTIC | Class 2, gel WEAK | Class 3, VISCOELASTIC gels | Class 4, VISCOUS gels |
|---|---|---|---|---|
| Tan δ | <<1 | <1 | >1 at low frequencies (e.g., 0.1 Hz), <1 at high frequencies (e.g., 10Hz) | >1 |
| Adhesivity A- | 0.05 - 0.06 N.mm | 0.1-0.5 N.mm | 0.5-2.5 N.mm | 0.01-0.5 N.mm |

in combination with suitable excipients and/or diluents, in which said mixture has:
a) at 1Hz: complex module G^{∗} comprised between 1 and 500 Pa and Tan δ comprised between 0.3 and 1
b) Adhesivity A- 0.08 - 5 N.mm and
c) the relative polysaccharides selected from Table 2 below

**TABLE 2**

| **INCI** | **RHEOLOGICAL PROPERTIES (class)** | **RANGE %** |
|---|---|---|
| Gellan Gum (high acylation) | ELASTIC GELS (1) | 0.1 -0.5 |
| Iota Carrageenan (Chondrus crispus) | ELASTIC GELS (1) | 0.20 - 0.75 |
| Xanthan Gum | WEAK GELS (2) | 0.5-1.5 |
| Sclerotium Gum | WEAK GELS (2) | 1.0 -2.0 |
| *Citrus lemon peel powder, Sclerotium gum* | WEAK GELS (2) | 1.0 - 2.0 |
| Succinoglycan | WEAK GELS (2) | 0.2-1.0 |
| Acacia Senegal Gum | WEAK GELS (2) | 0.75-1.5 |
| Modified potato starch | WEAK GELS (2) | 3.0 -5.0 |
| Astragalus Gummifer Gum | WEAK GELS (2) | 1.0 - 2.0 |
| Hydroxypropyl starch phosphate | WEAK GELS (2) | 5.0 -6.0 |
| Amorphophallus Konjac Root Extract | VISCOELASTIC GELS (3) | 0.75 - 1.25 |
| Hydroxypropyl Guar | VISCOELASTIC GELS (3) | 1.0 -2.5 |
| Caesalpinia spinosa gum | VISCOELASTIC GELS (3) | 1.0 - 2.0 |
| Ceratonia siliqua (carob) gum | VISCOELASTIC GELS (3) | 0.5 - 2.0 |
| Medium-high MW sodium hyaluronate | VISCOELASTIC GELS (3) | 0.01 -2.0 |
| Medium-high MW tamarindus indica seed polysaccharide | VISCOELASTIC GELS (3) | 0.01 -2.0 |
| C18-22 Hydroxyalkyl hydroxypropyl guar | VISCOELASTIC GELS (3) | 0.5 - 1.5 |
| *Cellulose gum, Algin* | VISCOELASTIC GELS (3) | 1.0 - 2.0 |
| Pectin | VISCOUS GELS (4) | 0.5 -6.0 |
| Tamarindus indica seed polysaccharide (low MW) | VISCOUS GELS (4) | 0.1 - 3.0 |
| Sodium hyaluronate (low MW) | VISCOUS GELS (4) | 0.5 - 2.0 |

These compositions are ideal for application to mucous membranes, especially oral or nasal, especially in terms of adhesion, ease of application and compliance, which makes them very effective for the desired therapeutic use.

These compositions are indicated for the treatment and prevention of viral and bacterial infections.

### DESCRIPTION OF THE FIGURES

Figures 1 and 2: Performance of the modules G' and G" as a function of the oscillation amplitude and the oscillation frequency of a Sclerotium gum-based sample,
Figures 3 and 4: Performance of the modules G' and G" as a function of the oscillation amplitude and the oscillation frequency of a Caesalpinia spinosa gum-based sample.
Figures 5 and 6: Performance of the modules G' and G" as a function of the oscillation amplitude and the oscillation frequency of a Ceratonia siliqua (carob) gum-based sample.
Figures 7 and 8: Performance of the modules G' and G" as a function of the oscillation amplitude and oscillation frequency of a sample based on the association 1 of the polymers Sclerotium Gum, Caesalpinia spinosa gum, Ceratonia siliqua (carob) gum.
Figure 9: Radar representation with the main texture parameters of the polymers present in Association 1.
Figures 10 and 11: Performance of the modules G' and G" as a function of the oscillation amplitude and the oscillation frequency of a Succinoglycan-based sample.
Figures 12 and 13: Performance of the modules G' and G" as a function of the oscillation amplitude and the oscillation frequency of a Carrageenan-based sample.
Figures 14 and 15: Performance of the modules G' and G" as a function of the oscillation amplitude (a) and the oscillation frequency (b) of a Tamarindus Indica Seed Polysaccharide-based sample.
Figures 16 and 17: Performance of the modules G' and G" as a function of the oscillation amplitude and the oscillation frequency of a sample based on association 2.

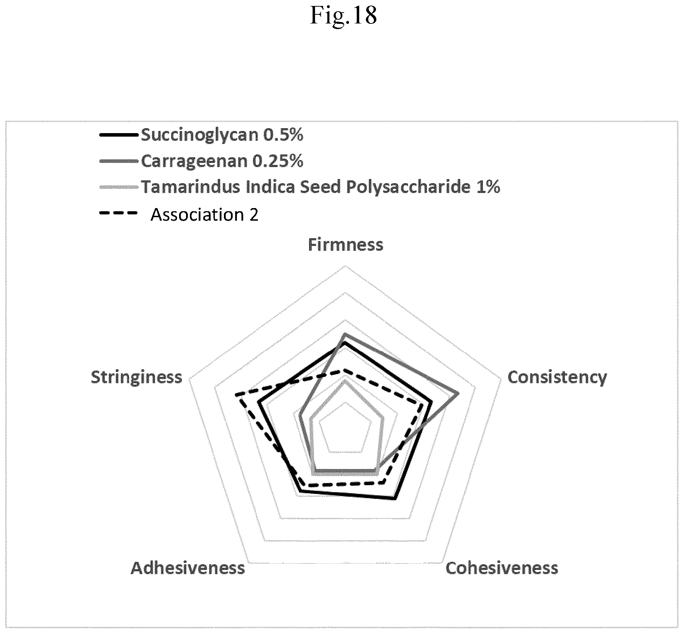

Figure 18: Radar representation with the main texture parameters of the polymers present in Association 2.
Figures 19 and 20: Performance of the modules G' and G" as a function of the oscillation frequency and texture parameters of Association 2 with and without the presence of bivalent salts.
Figures 21 and 22: Performance of the modules G' and G" as a function of the oscillation amplitude and the oscillation frequency of a Sclerotium gum-based sample,
Figures 23 and 24: Performance of the modules G' and G" as a function of the oscillation amplitude (a) and the oscillation frequency (b) of a Ceratonia siliqua (carob) gum-based sample.
Figures 25 and 26: Performance of the modules G' and G" as a function of the oscillation amplitude (a) and the oscillation frequency (b) of a Pectin-based sample.
Figures 27 and 28: Performance of the modules G' and G" as a function of the oscillation amplitude and oscillation frequency) of a sample based on the association of the polymers Sclerotium Gum, Ceratonia siliqua (carob) gum, Pectin.
Figure 29: Radar representation with the main texture parameters of the polymers present in Association 3.
Figures 30 and 31: Performance of the modules G' and G" as a function of the oscillation amplitude and oscillation frequency of a sample based on the association of the polymers Sclerotium Gum, Ceratonia siliqua (carob) gum, Pectin and Tamarindus indica seed polysaccharide
Figure 32: Radar representation with the main texture parameters of the polymers present in Association 4
Figures 33 and 34: Performance of the modules G' and G" as a function of the oscillation amplitude and oscillation frequency) of a sample based on the association of the polymers Sclerotium Gum, Caesalpinia spinosa gum and Pectin.
Figure 35: Radar representation with the main texture parameters of the polymers present in Association 5.
Figures 36 and 37: Performance of the modules G' and G" as a function of the oscillation amplitude and oscillation frequency of a sample of association 6 based on the association of the polymers Sclerotium Gum, Ceratonia siliqua (carob) gum, Caesalpinia spinosa gum and Tamarindus indica seed polysaccharide.
Figure 38: Radar representation with the main texture parameters of the polymers present in Association 6.
Figures 39 and 40: Performance of the modules G' and G" as a function of the oscillation amplitude (39) and the oscillation frequency (40) of a Hydroxypropyl guar gum-based sample
Figure 41 and 42: Performance of the modules G' and G" as a function of the oscillation amplitude (41) and the oscillation frequency (42) of a Sclerotium gum-based sample,
Figures 43 and 44: Performance of the modules G' and G" as a function of the oscillation amplitude (43) and the oscillation frequency (44) of a Cellulose gum, Algin-based sample

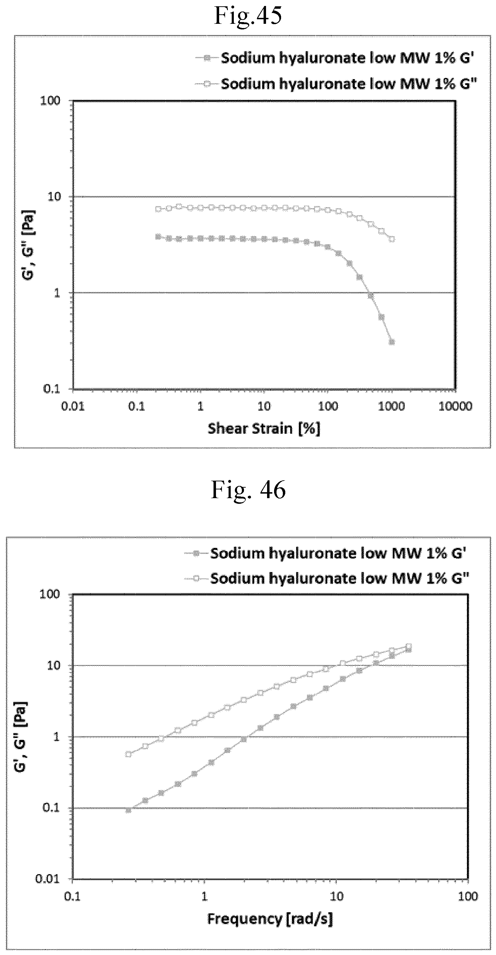

Figures 45 and 46: Performance of the modules G' and G" as a function of the oscillation amplitude (45) and the oscillation frequency (46) of a Sodium Hyaluronate LMW-based sample
Figures 47 and 48: Performance of the modules G' and G" as a function of the oscillation amplitude (47) and the oscillation frequency (48) of a sample based on the association of the polymers Hydroxypropyl guar gum, Sclerotium gum, Cellulose gum, algin and Sodium hyaluronate low MW
Figure 49: Radar representation with the main texture parameters of the polymers present in Association 7.
Figures 50-51: Performance of the modules G' and G" as a function of the oscillation amplitude (50) and the oscillation frequency (51) of a modified potato starch-based sample
Figures 52-53: Performance of the modules G' and G" as a function of the oscillation amplitude (52) and the oscillation frequency (53) of a Hydroxypropyl guar gum-based sample

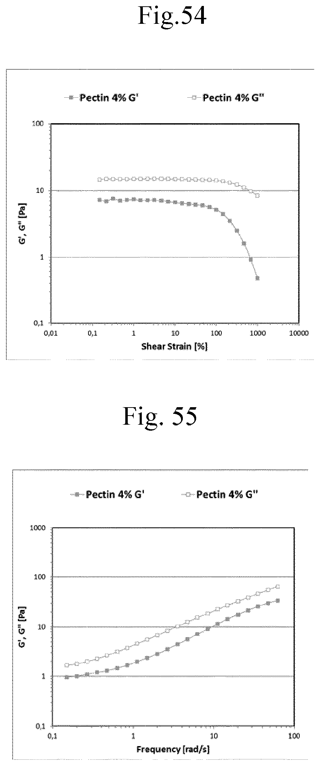

Figures 54-55: Performance of the modules G' and G" as a function of the oscillation amplitude (54) and the oscillation frequency (55) of a Pectin-based sample
Figures 56-57: Performance of the modules G' and G" as a function of the oscillation amplitude (56) and the oscillation frequency (57) of a Sodium hyaluronate high MW-based sample

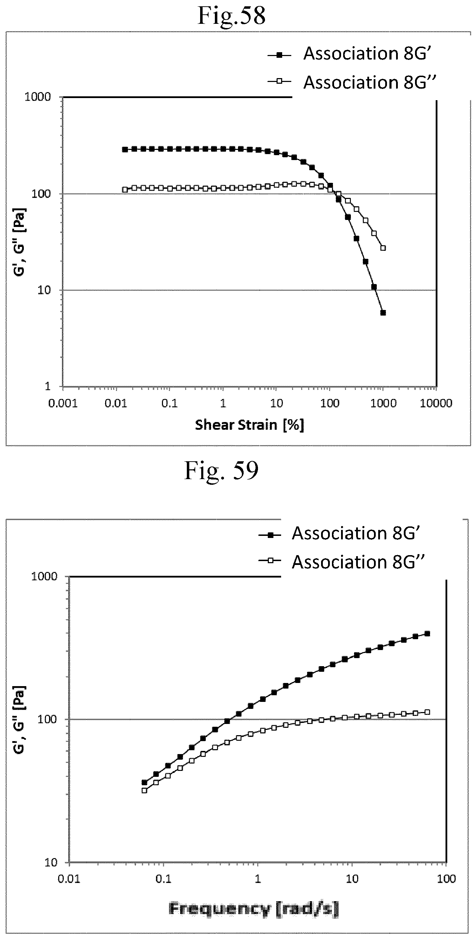

Figures 58-59: Performance of the modules G' and G" as a function of the oscillation amplitude (58) and the oscillation frequency (59) of a sample based on the association of the polymers Modified potato starch, Hydroxypropyl guar gum, Pectin, Sodium hyaluronate high MW

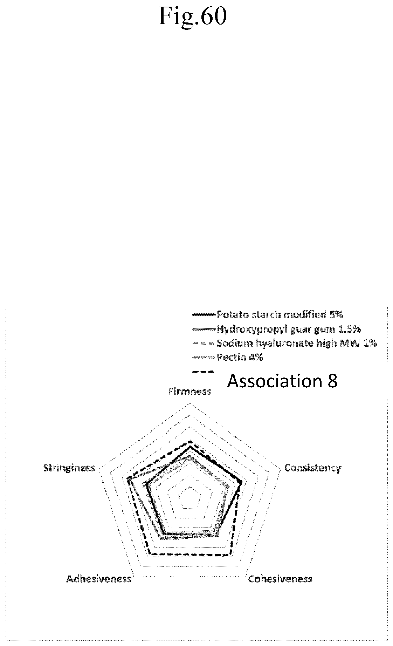

Figure 60: Radar representation with the main texture parameters of the polymers present in Association 8.

### DETAILED DESCRIPTION OF THE INVENTION

For the purposes of the present invention, the definitions "comprising" and "containing" provide for the possibility of further components in addition to those mentioned after said definition. Conversely, for the purposes of the present invention the definition "consisting of' excludes the possibility of further components other than those listed after said definition.

For the purposes of the present invention, G^{∗} of a material at a given frequency is the sum of the two modules G" + G' measured at said frequency, in which G" is the viscous module and is related to the dissipation energy, while G' is the elastic module and is related to the storage energy, while tan δ is the ratio G"/G'.

For the purposes of the present invention, δ is the phase shift angle if a strain is applied to said material.

When δ=90°, the material has the behaviour of an ideally viscous liquid.

When δ=0°, the material has the behaviour of an ideally elastic solid; in fact, in this case the application of a load generates an instantaneous strain (the two signals are in phase).

Instead, when 0< δ<90°, the material is viscoelastic.

When 0< δ<45°, the material has a solid-like behaviour; while when 45°< δ<90°, the material has a liquid-like behaviour.

For the purposes of the present invention, adhesivity A- was measured according to methods described in the literature (Dent. J. 2016, 4, 34; doi: 10.3390/dj4040034; International Journal of Cosmetic Science, 2016, 38, 389-398; Journal of Applied Polymer Science, Vol. 125, 180-188 (2012); Tropical Journal of Pharmaceutical Research August 2011; 10 (4): 393-401; Tamburic, Slobodanka and Sisson, Helen and Cunningham, Neil and Stevic, Milica (2017) Rheological and texture analysis methods for quantifying yield value and level of thixotropy. SOFW Journal, 143. pp.24-30).

The applicant, with the aim of finding polysaccharides which respond to the rheological properties and optimal adhesivity such as to make them particularly effective for the specific target, has selected and classified some polysaccharides in the four classes identified above in the above table 1 in the following types:
Class 1, ELASTIC gels
Class 2, WEAK gels
Class 3, VISCOELASTIC gels
Class 4, VISCOUS gels
to check whether they could be suitable for application to oral and nasal mucous membranes, if used as mono-components in mucosal compositions. The concentration ranges preferably used for each of these polysaccharides are set forth in the following Table 2

**Table 2**

| **INCI** | **RHEOLOGICAL PROPERTIES (class)** | **RANGE %** |
|---|---|---|
| Gellan Gum (high acylation) | ELASTIC GELS (1) | 0.1 -0.5 |
| Iota Carrageenan (Chondrus crispus) | ELASTIC GELS (1) | 0.20 - 0.75 |
| Xanthan Gum | WEAK GELS (2) | 0.5-1.5 |
| Sclerotium Gum | WEAK GELS (2) | 1.0 -2.0 |
| *Citrus lemon peel powder, Sclerotium gum* | WEAK GELS (2) | 1.0 - 2.0 |
| Succinoglycan | WEAK GELS (2) | 0.2-1.0 |
| Acacia Senegal Gum | WEAK GELS (2) | 0.75-1.5 |
| Modified potato starch | WEAK GELS (2) | 3.0 -5.0 |
| Astragalus Gummifer Gum | WEAK GELS (2) | 1.0 - 2.0 |
| Hydroxypropyl starch phosphate | WEAK GELS (2) | 5.0 -6.0 |
| Amorphophallus Konjac Root Extract | VISCOELASTIC GELS (3) | 0.75 - 1.25 |
| Hydroxypropyl Guar | VISCOELASTIC GELS (3) | 1.0 -2.5 |
| Caesalpinia spinosa gum | VISCOELASTIC GELS (3) | 1.0 - 2.0 |
| Ceratonia siliqua (carob) gum | VISCOELASTIC GELS (3) | 0.5 - 2.0 |
| Medium-high MW sodium hyaluronate | VISCOELASTIC GELS (3) | 0.01 -2.0 |
| Medium-high MW tamarindus indica seed polysaccharide | VISCOELASTIC GELS (3) | 0.01 -2.0 |
| C18-22 Hydroxyalkyl hydroxypropyl guar | VISCOELASTIC GELS (3) | 0.5 - 1.5 |
| *Cellulose gum, Algin* | VISCOELASTIC GELS (3) | 1.0 - 2.0 |
| Pectin | VISCOUS GELS (4) | 0.5 -6.0 |
| Tamarindus indica seed polysaccharide (low MW) | VISCOUS GELS (4) | 0.1 - 3.0 |
| Sodium hyaluronate (low MW) | VISCOUS GELS (4) | 0.5 - 2.0 |

All these polysaccharides, depending on the class to which they belong, have the rheology and adhesivity features shown in the aforementioned Table 1: such features occur within the concentration range indicated in Table 2.

However, no individually-used polysaccharide has all the properties necessary to ensure the optimal technical requirements and film-forming/adhesive properties for application to oral or nasal mucous membranes.

In fact, the applicant has found that the ideal and optimal features of such polysaccharides are:
a) at 1 Hz: complex module G^{∗} comprised between 1 and 500 Pa and Tan δ comprised between 0.3 and 1;
b) Adhesivity A- 0.08 - 5 N.mm.

These features are obtained by mixing at least 3 polysaccharides belonging to at least 2 of the aforesaid classes 1, 2, 3 and 4.

The mucosal compositions administered in the oral cavity preferably have
a) at 1 Hz: G^{∗} is comprised between 150 and 500 Pa and Tan δ is comprised between 0.3 and 1
b) adhesivity: A- is comprised between 0.8 and 5 N.mm,

The nasally administrable mucosal compositions are preferably those having:
c) at 1 Hz: G^{∗} between 1 and 150 Pa and Tan δ between 0.4 and 1 and
d) adhesivity A- between 0.08 and 1.2 N.mm.

Examples of preferred associations include, but are not limited to:

### Association 1:

**Polymers used (3 polymers including 1 weak gel and 2 viscoelastic gels):**

| Polysaccharide (class) | % used in the association: |
|---|---|
| Sclerotium gum (2) | 1% |
| Caesalpinia spinosa gum (3) | 1% |
| Ceratonia siliqua (carob) gum (3) | 0.5% |

The Frequency Sweep (FS) analysis, also defined as the mechanical spectrum of the material, allows the qualitative-quantitative characterization of the viscoelastic properties: G' (elastic module) and G" (viscous module) and is carried out by applying a constant oscillation amplitude, comprised in the region of linear viscoelasticity, varying the oscillation frequency. This analysis therefore allows to "photograph" the features of the structure which the polymer determines by interacting with the aqueous solvent in which it is dispersed.

The analyses were performed by Rheoplus Anton Paar MCR 101 rotational rheometer at a constant temperature of 23±0.05°C using a flat/smooth cone geometry sensor.

In the analysis of oscillation amplitude (AS), each sample was analysed as a function of the fixed frequency strain (1Hz/0.1Hz) to determine the linear viscoelasticity zone of the material, a strain range, within which the sample does not undergo irreversible changes.

### RHEOLOGICAL CHARACTERIZATION OF Sclerotium gum

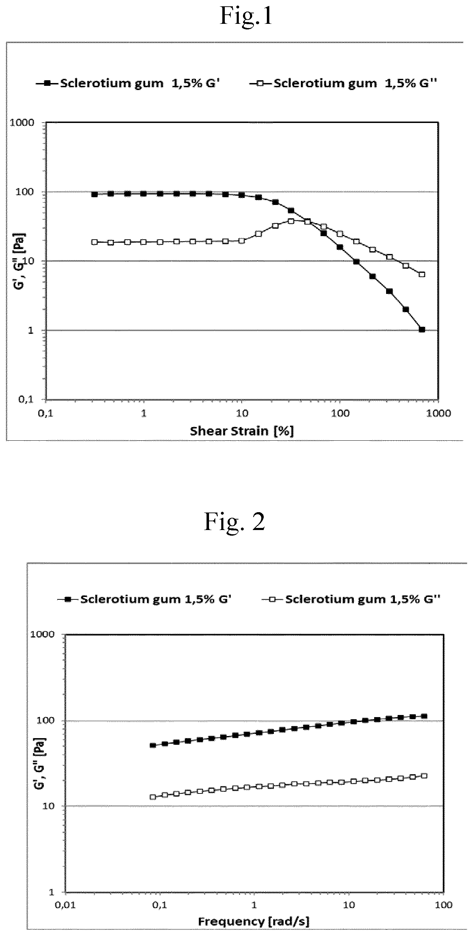

The Sclerotium gum-based gel has rheological behaviour, obtained as a function of the oscillation frequency at constant strain amplitude (frequency sweep) typical of a weak gel, i.e., it has the following features:
- the elastic component G' is always higher than the viscous component G" throughout the frequency range investigated;
- the two modules have a trend parallel to the axis of the abscissae, i.e., did not significantly vary with the variation of the frequency;
- the absolute values of the two modules are comprised between the second and the third set of ten (medium-low quantitative rheological response). (Fig. 2)

### RHEOLOGICAL CHARACTERIZATION OF Caesalpinia spinosa gum

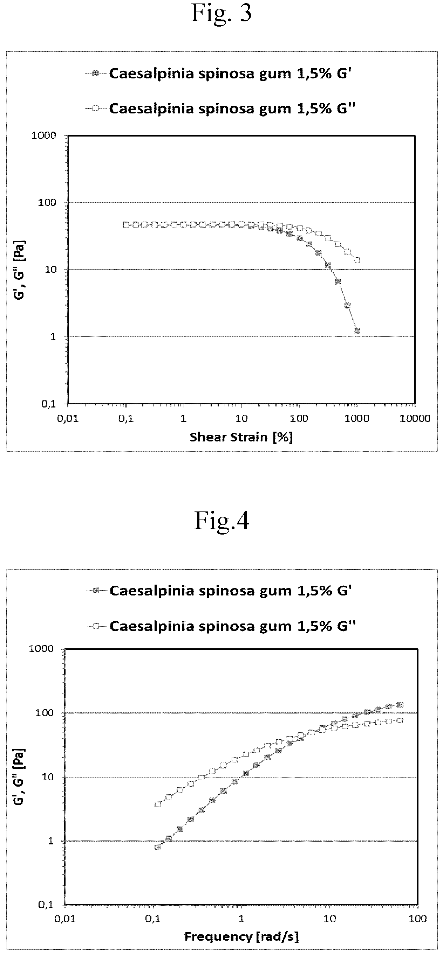

The Caesalpinia spinosa gum-based sample has viscoelastic behaviour, in which the elasticity at high frequencies prevails (the elastic module G' is greater than the viscous module G"), while at low frequencies the situation is reversed with the prevalence of the viscous character (the module G" greater than the module G') (Fig. 4). The crossing point between the two modules is located at a frequency of 6.38 rad/s (Fig.4).

### RHEOLOGICAL CHARACTERIZATION OF Ceratonia siliqua (carob) gum

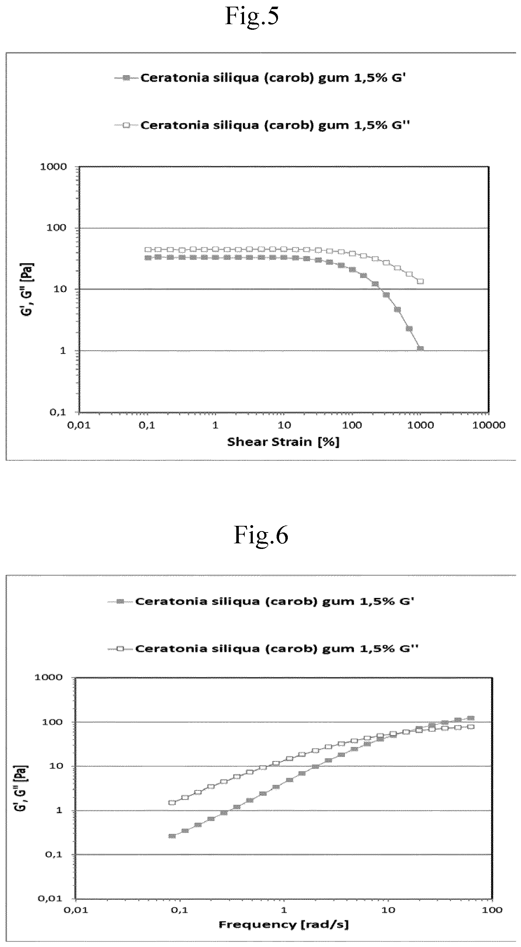

A Ceratonia siliqua gum-based gel has a gelled structure similar to a Caesalpinia spinosa gum gel, therefore of viscoelastic type but, with the same concentration (e.g. 1.5%) the viscous character is slightly higher (viscous module G" higher than the elastic module G' visible in the measurement in sweep amplitude at constant frequency (Fig. 5) and crossing point between the higher frequency modules (14.9 rad/s) in the FS measurement at constant strain amplitude (Fig. 6).

### RHEOLOGICAL AND TEXTURE CHARACTERIZATION OF ASSOCIATION 1 (3 polymers including 1 weak gel and 2 viscoelastic gels)

Association 1 of the 3 polymers in specific ratios involves the formation of a gelled structure characterized by viscoelastic properties which are however different from those of each single polymer used.

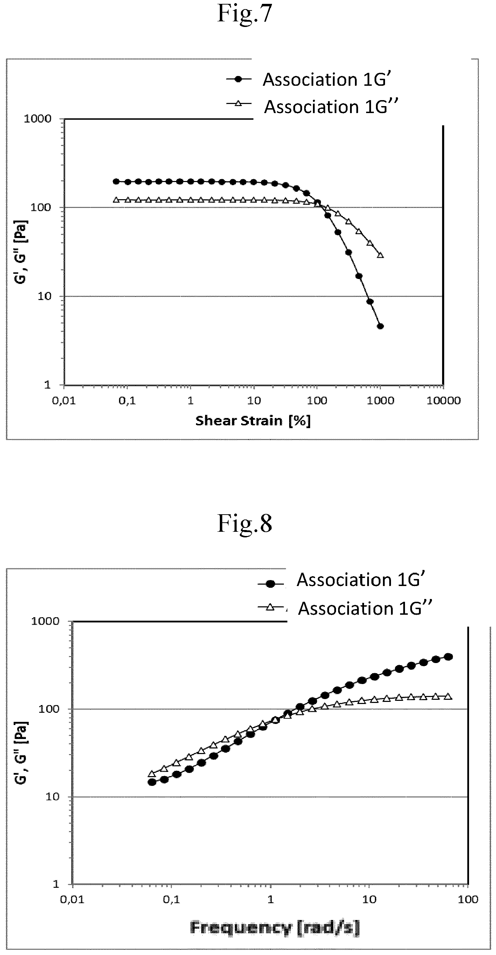

The association, by virtue of the presence of the polymer with weak gel properties with a predominantly elastic character, has an elastic component G' higher than that of the two viscoelastic rubbers used in the mixture: G' is always higher than G" in the measurement in sweep amplitude at constant frequency (Fig.7) and the cross-over point between the modules in the mechanical spectrum obtained at constant strain amplitude falls at lower frequencies (1.12 rad/s) (Fig. 8)

The absolute values of the two modules in sweep amplitude (figure 7 fall in the third set of ten, medium-low quantitative rheological response).

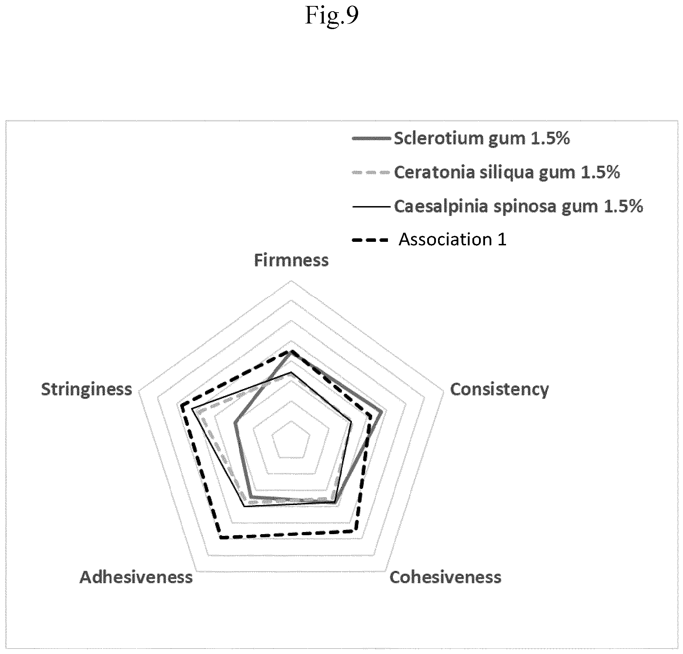

Comparing the application features of the individual polymers and the association through texture analysis measurements, a significant increase in the consistency and adhesivity features of the gel is observed (Fig.9).

A product is thus obtained with optimal features for adhesion on the oral mucosa.
**G* (at 1Hz): 230 Pa**
**Tan** δ **(at 1Hz): 0.6**
**Adhesivity (A-): 1.9 N.mm**

### Association 2:

**Polymers used (3 polymers including 1 weak gel, 1 elastic gel and 1 viscous gel):**

| Polysaccharide (class) | % used in the association: |
|---|---|
| Succinoglycan (2) | 0.2% |
| Carrageenan (iota carrageenan)(1) | 0.2% |
| Tamarindus Indica Seed Polysaccharide (4) | 0.2% |

### RHEOLOGICAL CHARACTERIZATION OF Succinoglycan

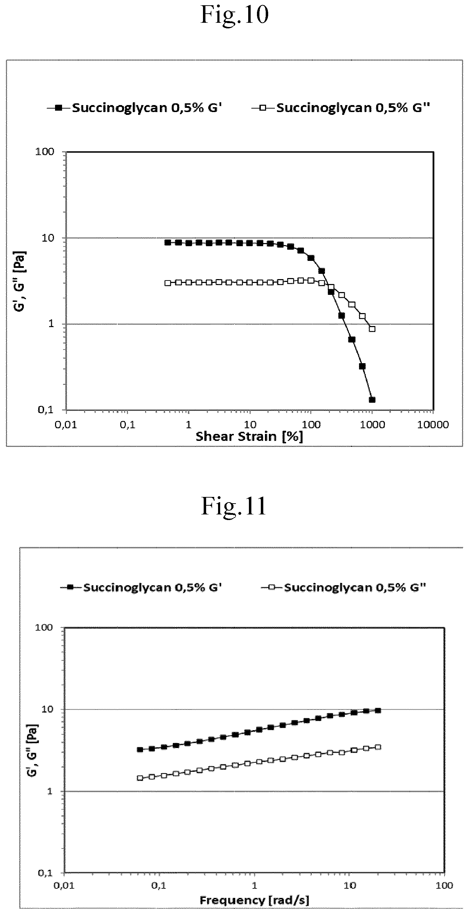

The Succinoglycan-based gel has rheological behaviour, obtained as a function of the oscillation frequency at constant strain amplitude (frequency sweep) typical of a weak gel, i.e., it has the following features:
- the elastic component G' is always higher than the viscous component G" throughout the frequency range investigated (Fig. 11);
- the two modules have a trend parallel to the axis of the abscissae, i.e., did not significantly vary with the variation of the frequency (Fig. 11);
- the absolute values of the two modules are comprised in the second set of ten (low quantitative rheological response). (Fig. 11).

### RHEOLOGICAL CHARACTERIZATION OF Carrageenan (iota carrageenan)

The gelled system based on iota carrageenan is characterized by the prevalence of an elastic character: G' (elastic module) is much higher than the viscous module G" in terms of both oscillation amplitude and frequency.

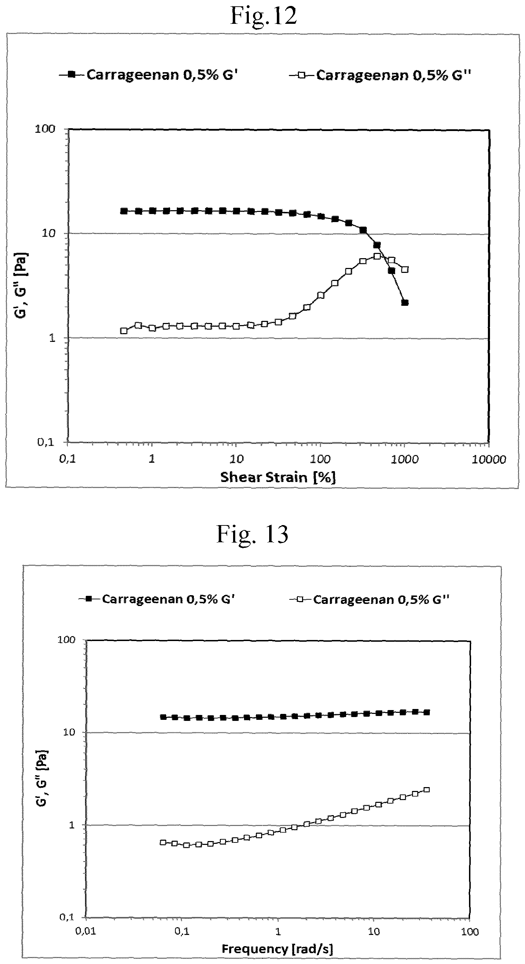

Iota carragenaan forms resistant gels because, to induce irreversible structural alterations, it is necessary to impart very large strains (this is observed from the very wide linear viscoelasticity range in sweep amplitude (Fig. 12) even if the quantitative responses of the modules fall in the first set of ten (low quantitative rheological response).

### RHEOLOGICAL CHARACTERIZATION OF Tamarindus Indica Seed Polysaccharide (low molecular weight)

The polymer forms a structured solution of predominantly viscous character.

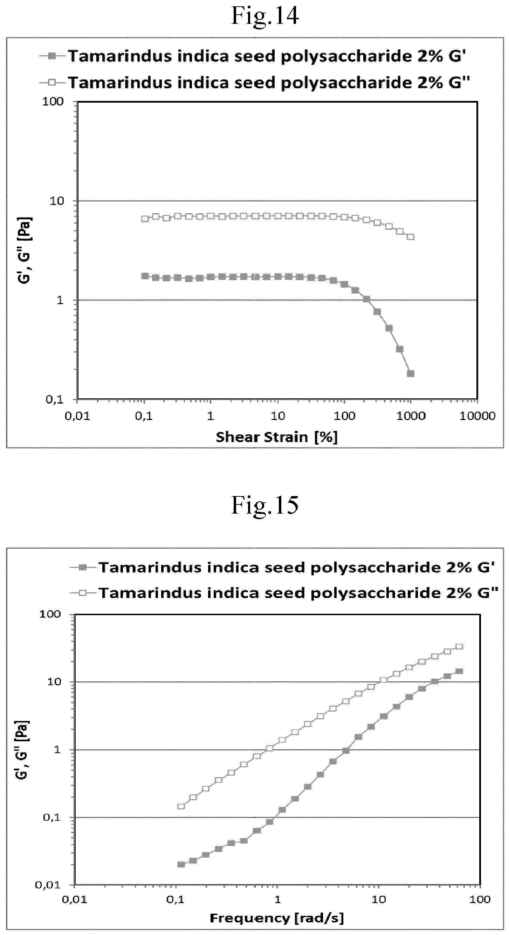

G", viscous module, is always higher than the elastic component G' both in SWEEP AMPLITUDE (Fig. 14) and as a function of frequency (Fig. 15). Both modules are very sensitive to the frequency variation (trends decrease significantly as the applied frequency decreases) and the quantitative responses are low (G" values fall in the second set of ten).

### RHEOLOGICAL CHARACTERIZATION AND TEXTURE OF ASSOCIATION 2: (3 polymers including 1 weak gel, 1 elastic gel and 1 viscous gel)

### 0.2% Succinoglycan, 0.2% Carrageenan, 0.2% Tamarindus Indica Seed Polysaccharide

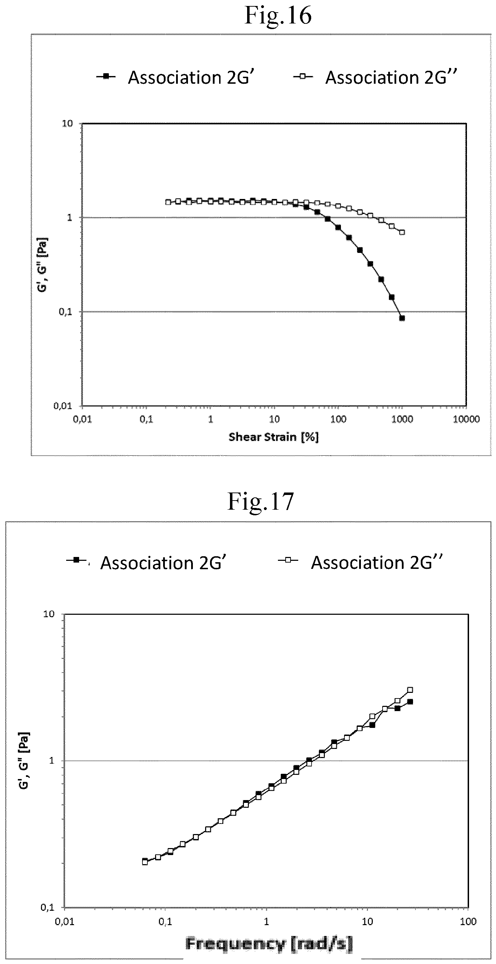

The association of the 3 polymers in this specific relationship forms a very fluid structure (low quantitative rheological response) in which the elastic and viscous components which have similar and overlapping trends in both Sweep Amplitude (Fig. 16) and Sweep Frequency (Fig. 17) are present in equal measure.

By associating the polymers together, a fluid system is obtained, by virtue of the presence of liquid-viscous components (Tamarindus indica seed polysaccharide), which maintains the adhesivity features of the Succinoglycan polymer present in the formulation. Such features are consistent with those of a product for nasal application.
**G* (at 1Hz): 2.1 Pa**
**Tan** δ **(at 1Hz): 1**
**Adhesivity (A-): 0.1 N.mm**

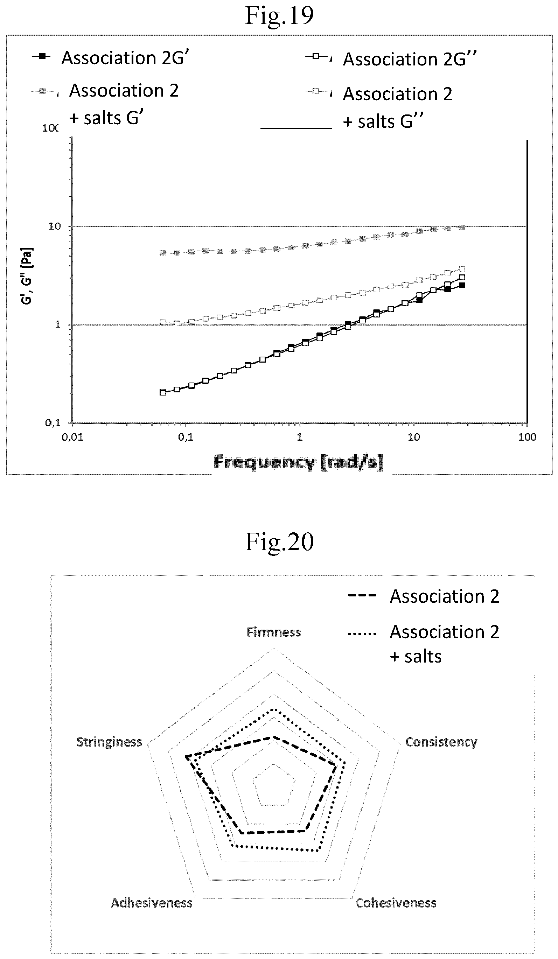

If divalent salts in the amounts present in the nasal mucosa (Calcium chloride and Magnesium sulphate) are added to ASSOCIATION 2, gelling of the structure is observed with increased elasticity, consistency, firmness and adhesivity of the gel. (Figures 19 and 20)

After being dispensed and once in contact with the mucosa, the product increases its structure, allowing a better adhesivity and prolonged protective film-forming effect (barrier effect).

### Association 3:

**Polymers used (3 polymers including 1 weak gel, 1 viscoelastic gel and 1 viscous gel):**

| Polysaccharide (class) | % weight used in the association |
|---|---|
| Sclerotium gum (2) | 1.25% |
| Ceratonia siliqua (carob) gum (3) | 1% |
| Pectin (4) | 2% |

The rheological analyses under oscillatory conditions of the polymers forming Association 3 are shown below.

### RHEOLOGICAL CHARACTERIZATION OF Sclerotium gum

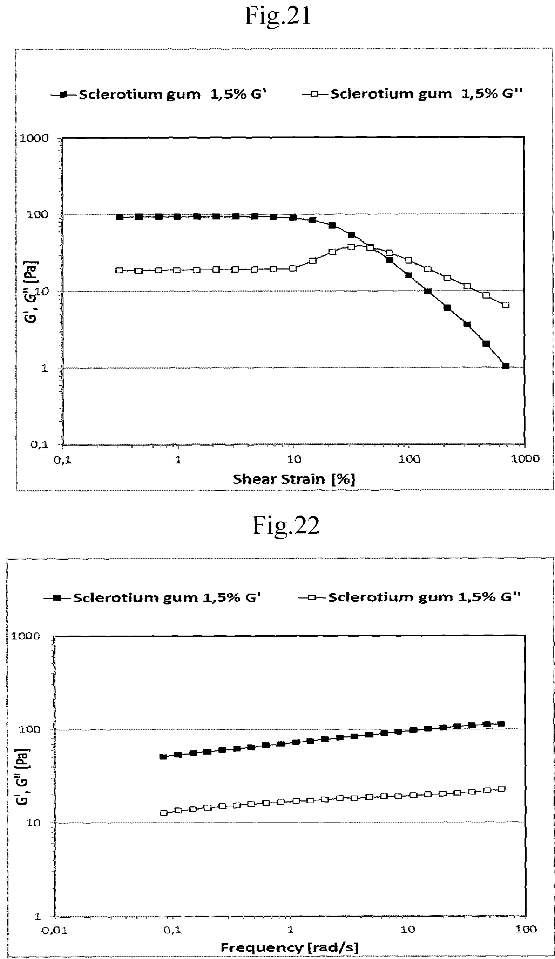

The Sclerotium gum-based gel has rheological behaviour, obtained as a function of the oscillation frequency at constant strain amplitude (frequency sweep) typical of a weak gel, i.e., it has the following features:
- the elastic component G' is always higher than the viscous component G" throughout the frequency range investigated;
- the two modules have a trend parallel to the axis of the abscissae, i.e., did not significantly vary with the variation of the frequency;
- the absolute values of the two modules are between the second and the third set of ten (medium-low quantitative rheological response) (Fig. 22).

### RHEOLOGICAL CHARACTERIZATION OF Ceratonia siliqua (carob) gum

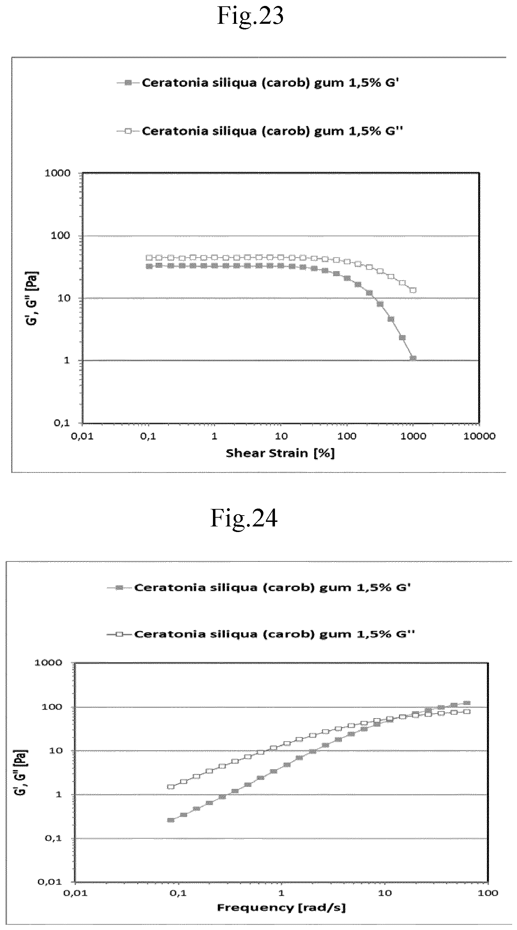

A Ceratonia siliqua gum-based gel has a gelled structure similar to a Caesalpinia spinosa gum gel, therefore of viscoelastic type but, with the same concentration (e.g. 1.5%) the viscous character is slightly higher (viscous module G" higher than the elastic module G' visible in the measurement in oscillation amplitude at constant frequency (Fig. 23) and crossing point between the higher frequency modules (14.9 rad/s) in the oscillatory frequency measurement at constant strain amplitude (Fig.24).

### RHEOLOGICAL CHARACTERIZATION OF Pectin

The polymer forms a structured solution of predominantly viscous character.

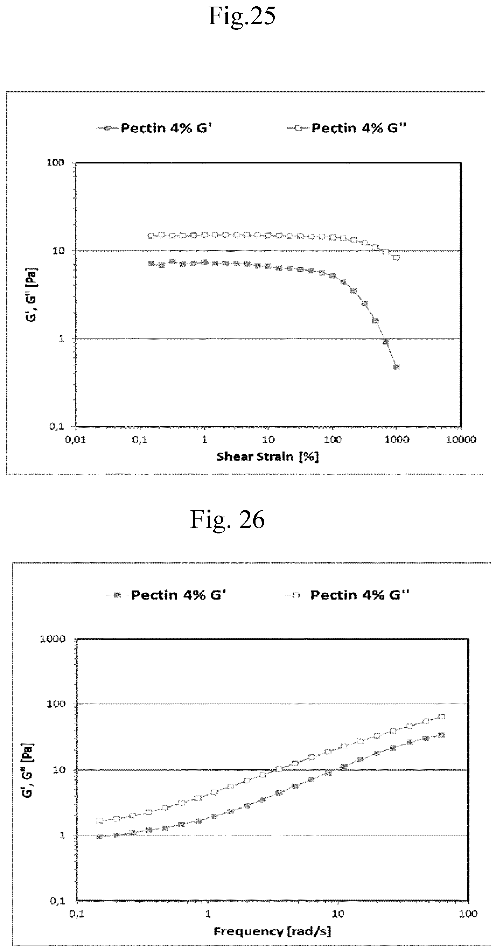

G", viscous module, is always higher than the elastic component G' in both oscillatory amplitude (Fig.25) and as a function of oscillatory frequency (Fig.26). Both modules are sensitive to frequency variations (trends decrease as the applied frequency decreases) and the quantitative responses are low.

### RHEOLOGICAL CHARACTERIZATION AND TEXTURE OF ASSOCIATION 3: (3 polymers including 1 weak gel, 1 viscoelastic and 1 viscous)

### Sclerotium Gum 1.25%, Ceratonia siliqua (carob) gum 1%, Pectin 2%,

The association of the three polymers in specific ratios involves the formation of a gelled structure characterized by unique properties and different from those of each single polymer used.

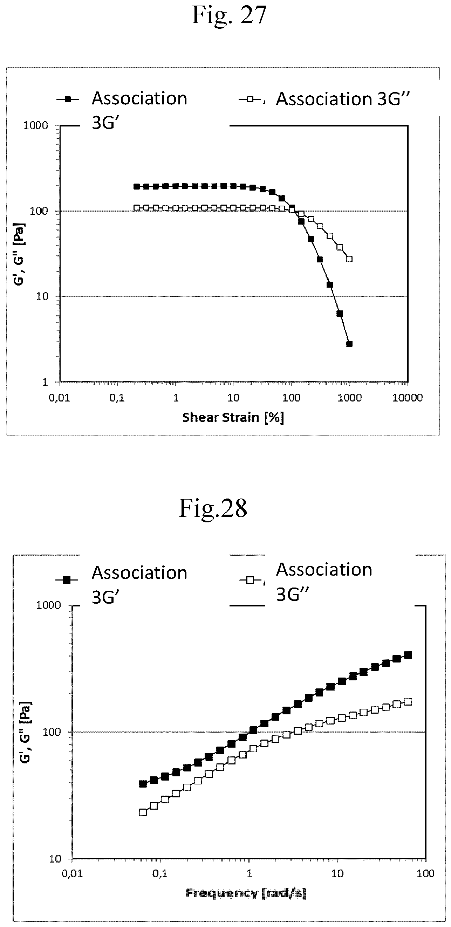

The predominantly viscous contribution of Pectin and Ceratonia siliqua gum on the weak gel structure of Sclerotium gum gives rise to a structure with a predominantly elastic character. The elastic module G' remains higher than the viscous module G" throughout the frequency range (Fig.28), but the module varies strongly with the frequency, thus placing Association 3 between the viscoelastic materials and the weak gels.

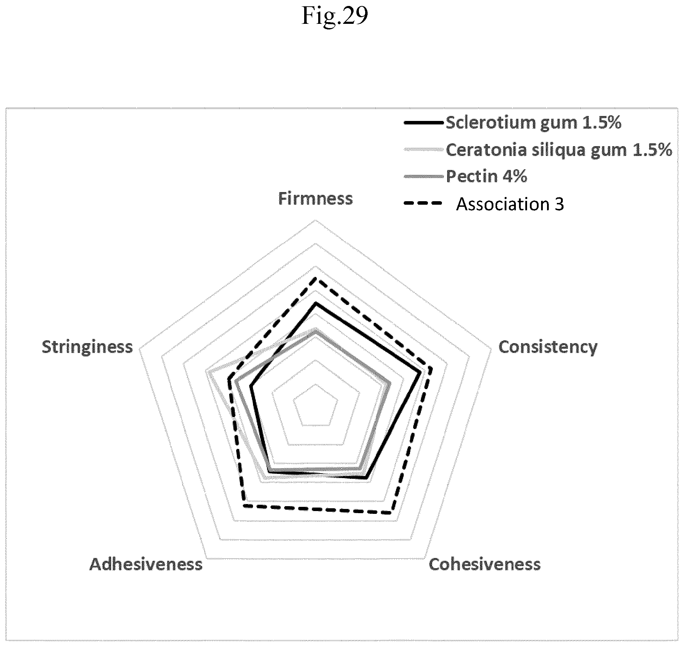

Comparing the application features of the individual polymers and the association through texture analysis measures, a significant increase in the adhesivity, cohesiveness, consistency and firmness of the gel is observed. (Fig.29)

Thus, a product with features suitable for application on the oral mucosa is obtained, features which could not be satisfied with the use of a single polymer.
**G* (at 1Hz): 224 Pa**
**Tan** δ **(at 1Hz): 0.6**
**Adhesivity (A-): 1.5 N.mm**

### Association 4:

**Polymers used (4 polymers including 1 weak gel, 1 viscoelastic gel, 2 viscous gels):**

| Polysaccharide (class) | % used in the association: |
|---|---|
| Sclerotium gum (2) | 1.25 |
| Ceratonia siliqua (carob) gum (3) | 1 |
| Pectin (4) | 0.5 |
| Tamarindus indica seed polysaccharide (4) | 0.5 |

For the characterization of the individual polymers, please refer to the previous associations.

### RHEOLOGICAL CHARACTERIZATION AND TEXTURE OF ASSOCIATION 4: 4 polymers including 1 weak gel, 1 viscoelastic gel, 2 viscous gels

The association of the 4 polymers, including a polymer which forms weak gels, a viscoelastic gel and 2 viscous gels, in specific ratios, involves the formation of a gelled structure characterized by unique properties and different from those of each single polymer used.

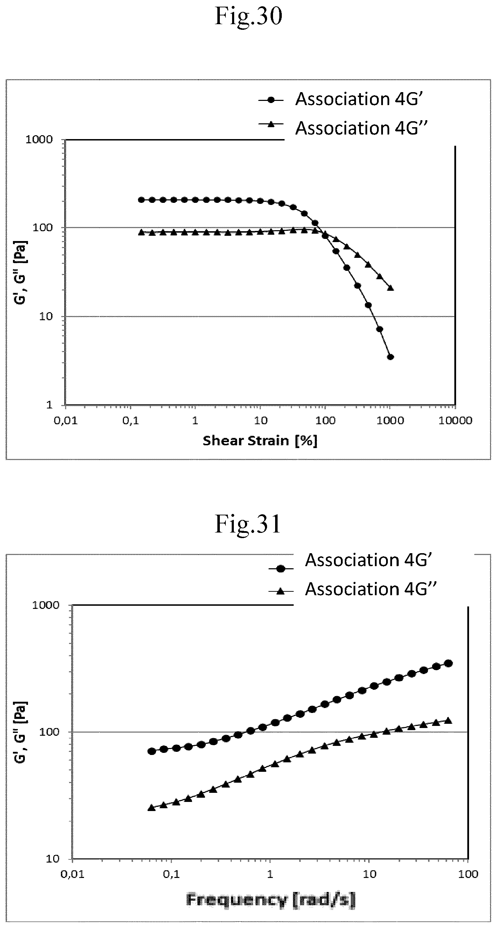

The predominantly viscous contribution of Pectin and Tamarindus Indica Seed Polysaccharide on the weak gel structure of Sclerotium gum gives rise to a structure with a predominantly elastic character. The elastic module G' remains higher than the viscous module G" throughout the frequency range but the module varies strongly with the frequency, thus placing Association 4 between the viscoelastic materials and the weak gels (Fig.31)

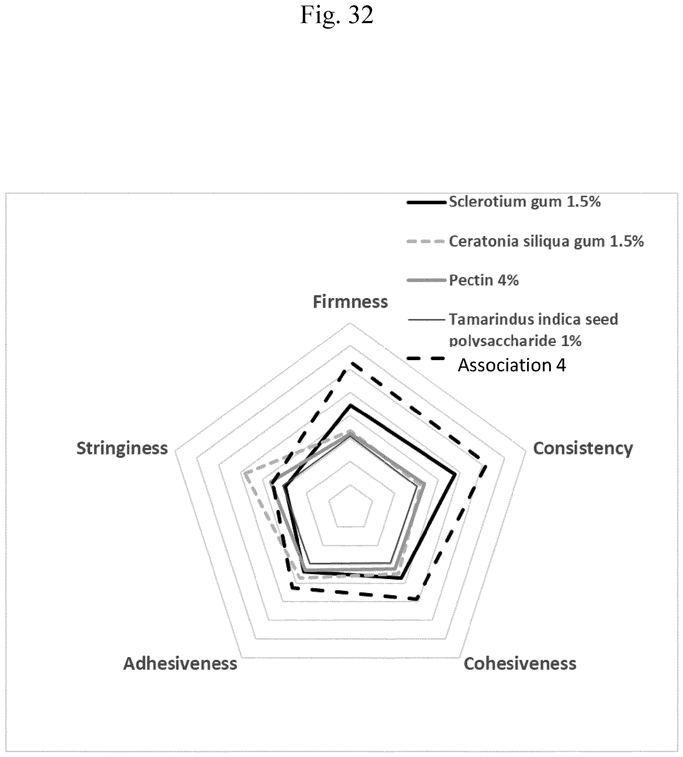

Comparing the application features of the individual polymers and the association through texture analysis measures, a significant increase in the consistency and firmness of the gel is observed. (Fig.32)

The parameters fall among the products considered suitable for application on oral mucosa:
**G* (at 1Hz): 226 Pa**
**Tan** δ **(at 1Hz): 0.5**
**Adhesivity (A-): 0.9 N.mm**

### Association 5:

**Polymers used: 3 polymers including 1 weak gel, 1 viscoelastic gel, 1 viscous gel**

| Polysaccharide (class) | % used in the association: | |
|---|---|---|
| Sclerotium gum (2) | 1.25 | |
| Caesalpinia spinosa gum (3) | 1% | 5 |
| Pectin (4) | 1% | |

For the characterization of the individual polymers, please refer to the previous associations.

### CHARACTERIZATION OF ASSOCIATION 5

The association of the 3 polymers in specific ratios also involves in this case the formation of a gelled structure characterized by unique and balanced properties in which the rheological properties of the polymers are modulated.

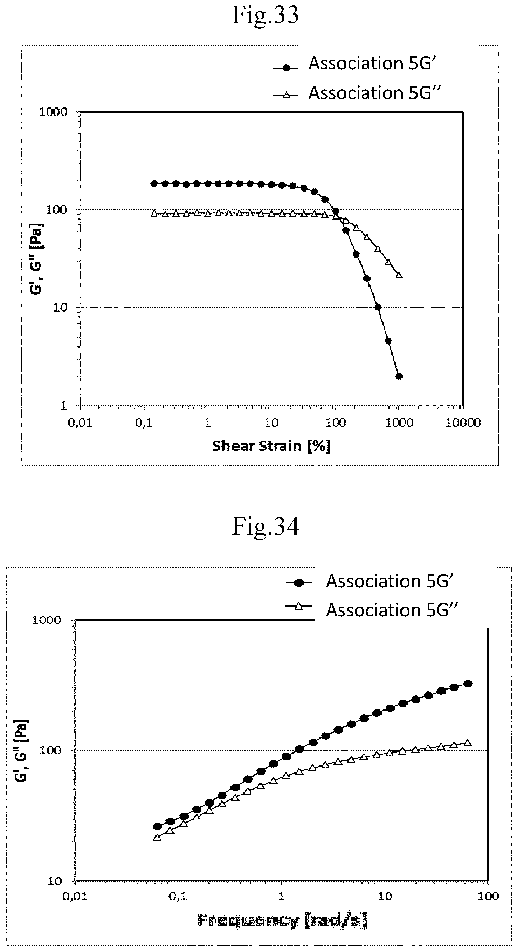

The rheological features of Association 5 are the result of the association of a weak gel (Sclerotium gum), a viscoelastic gel (Caesalpinia spinosa gum) and a viscous polymer (Pectin) but are not attributable to the features of any of these 3 polymers: the elastic module G' remains higher than the viscous G" throughout the frequency range but the modules vary strongly with the frequency. (Fig. 34)

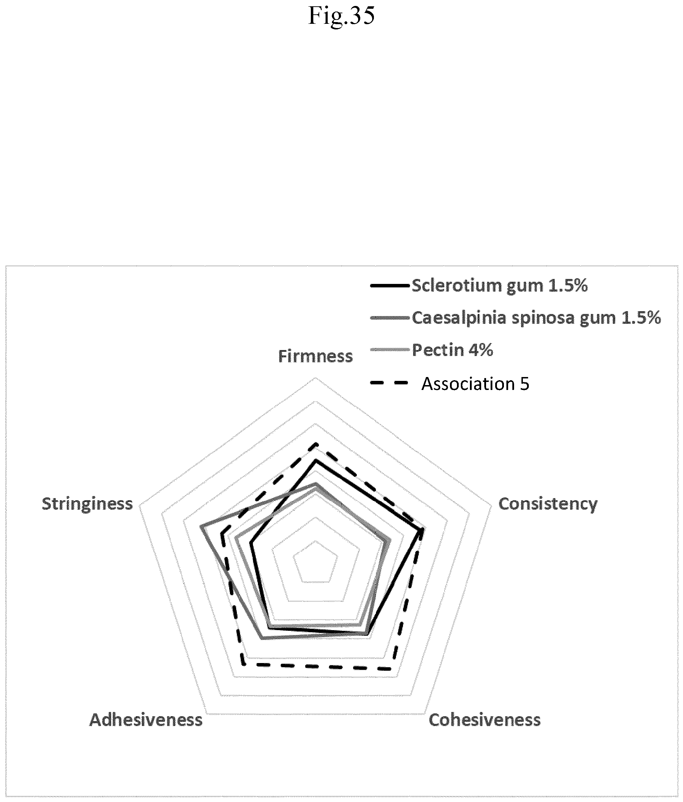

Comparing the application features of the individual polymers and the association through texture analysis measures, a significant increase in the consistency and firmness of the gel is observed. (Fig.35)

The parameters fall among the products considered suitable for application on oral mucosa:
**G* (at 1Hz): 208 Pa**
**Tan δ(at 1Hz): 0.5**
**Adhesivity (A-): 1.55 N.mm**

### Association 6:

**Polymers used: 4 polymers including 1 weak gel, 2 viscoelastic gels, 1 viscous gel**

| Polysaccharides (class) | % used in the association: |
|---|---|
| Sclerotium gum (2) | 1 |
| Ceratonia siliqua (carob) gum (3) | 0.5 |
| Caesalpinia spinosa gum (3) | 1 |
| Tamarindus indica seed polysaccharide (4) | 0.1% |

For the characterization of the individual polymers, please refer to the previous associations.

### CHARACTERIZATION OF ASSOCIATION 6

The association of the 4 polymers in specific ratios involves the formation of a gelled structure characterized by unique properties and different from those of each single polymer used.

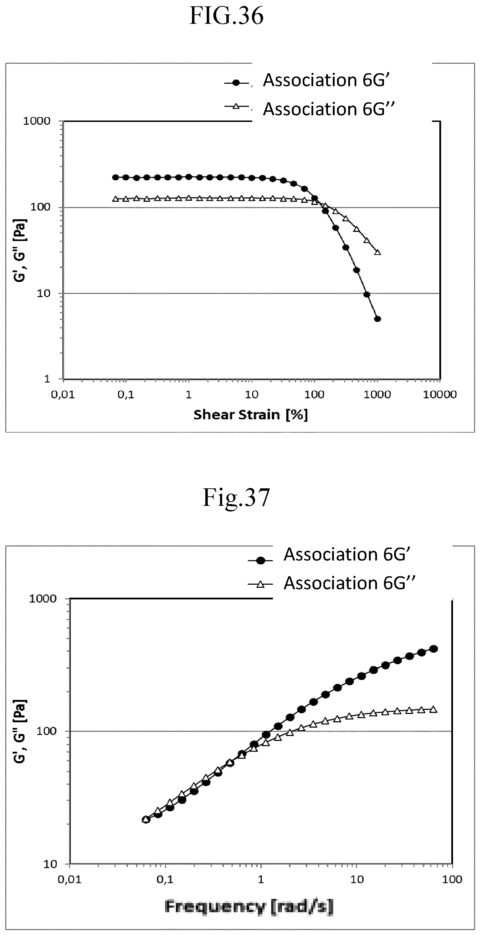

Sclerotium gum gives elasticity to the viscoelastic structure of the Ceratonia siliqua gum and Caesalpinia spinosa gum rubbers and to the viscous structure of *Tamarindus indicates seed polysaccharide*: in fact, the viscoelastic structure deriving from the association has a crossing point shifted towards low frequencies indicating a greater elastic character (Fig.36).

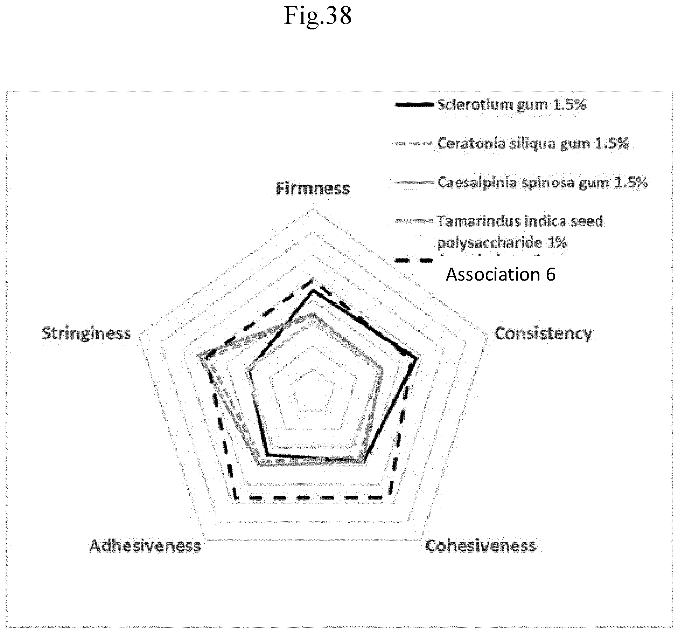

Comparing the application features of the individual polymers and the association through texture analysis measures, a significant increase in the cohesiveness and adhesivity features of the gel with respect to the individual polymers is observed. (figure 38)

The parameters fall among the products considered suitable for application on oral mucosa:
**G* (at 1Hz): 255 Pa**
**Tan d (at 1Hz): 0.6**
**Adhesivity (A-): 1.75 N.mm**

### Association 7:

**Polymers used (4 polymers including 1 weak gel, 2 viscoelastic gels and 1 viscous gel):**

| Polysaccharide (class) | % used in the association: | |
|---|---|---|
| Hydroxypropyl guar gum (3) | 1% | |
| Sclerotium gum (2) | 1% | |
| Cellulose gum, algin (3) | 1% | |
| Sodium hyaluronate low MW (4) | 0.5% | 20 |

The rheological analyses under oscillatory conditions of the polymers forming Association 7 are shown below.

### RHEOLOGICAL CHARACTERIZATION OF Hydroxypropyl guar gum

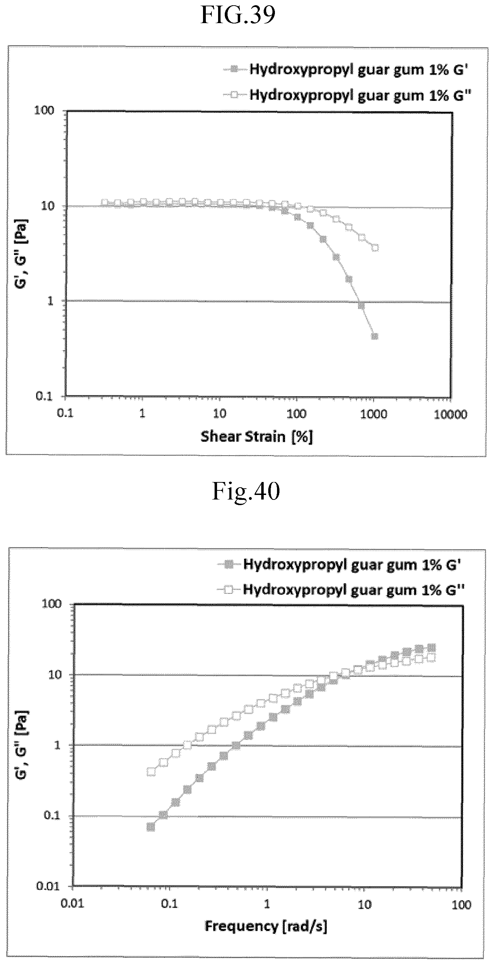

The Hydroxypropyl Guar gum-based sample has viscoelastic behaviour, in which the elasticity at high frequencies prevails (the elastic module G' is greater than the viscous module G"), while at low frequencies the situation is reversed with the prevalence of the viscous character (the module G" greater than the module G') (figure 40). The crossing point between the two modules is located at a frequency of 8.38 rad/s.

### RHEOLOGICAL CHARACTERIZATION OF Sclerotium gum

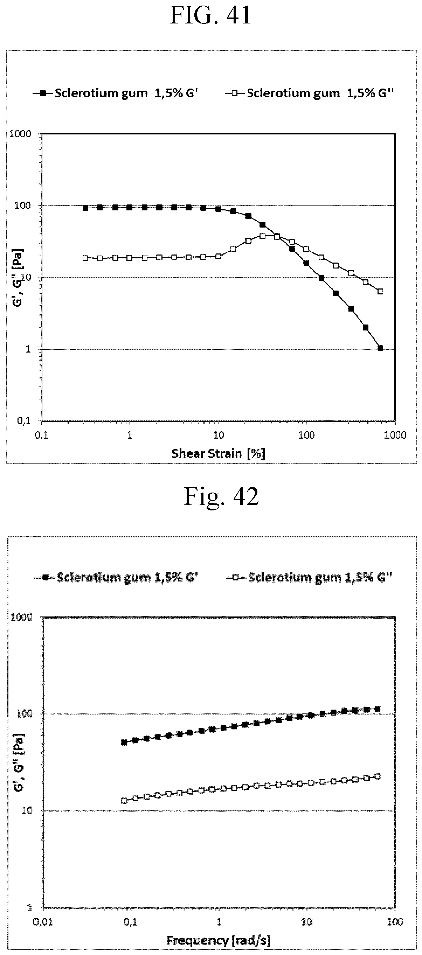

The Sclerotium gum-based gel has rheological behaviour, obtained as a function of the oscillation frequency at constant strain amplitude (frequency sweep) typical of a weak gel, i.e., it has the following features:
- the elastic component G' is always higher than the viscous component G" throughout the frequency range investigated;
- the two modules have a trend parallel to the axis of the abscissae, i.e., did not significantly vary with the variation of the frequency;
- the absolute values of the two modules are between the second and the third set of ten (medium-low quantitative rheological response). (figure 42)

### RHEOLOGICAL CHARACTERIZATION OF Cellulose gum, algin

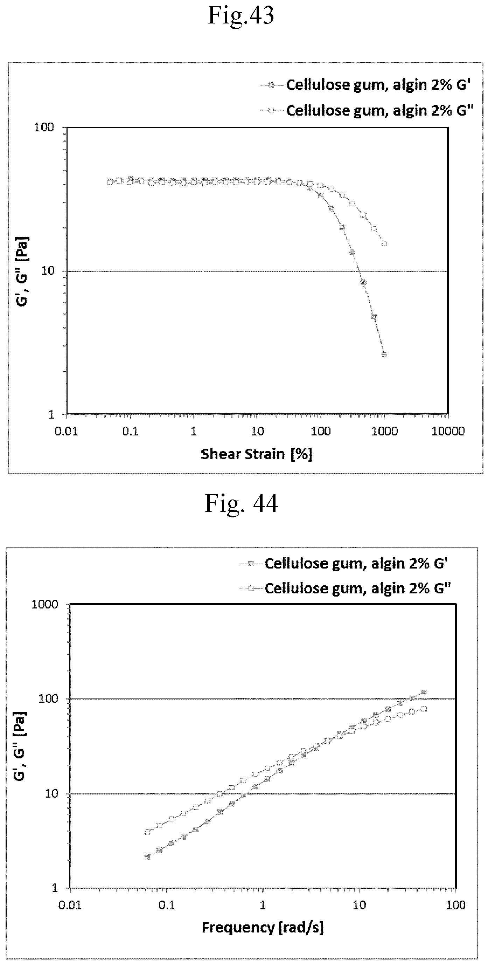

The Cellulose gum, Algin-based sample has viscoelastic behaviour, in which the elasticity at high frequencies prevails (the elastic module G' is greater than the viscous module G"), while at low frequencies the situation is reversed with the prevalence of the viscous character (the module G" greater than the module G') (fig.44). The crossing point between the two modules is located at a frequency of 4.71 rad/s.

### RHEOLOGICAL CHARACTERIZATION OF Sodium Hyaluronate LMW

The polymer forms a structured solution of predominantly viscous character. G", viscous module, is always higher than the elastic component G' both in As and as a function of the frequency. Both modules are very sensitive to the frequency variation (trends decrease significantly as the applied frequency decreases).

### RHEOLOGICAL CHARACTERIZATION AND TEXTURE OF ASSOCIATION 7:

### (4 polymers including 1 weak gel, 2 viscoelastic gels and 1 viscous gel)

Hydroxypropyl guar gum 1%, Sclerotium gum 1%, Cellulose gum, algin 1%, Sodium hyaluronate low MW 0.5%

The association of the 4 polymers in specific ratios involves the formation of a gelled structure characterized by unique properties and different from those of each single polymer used.

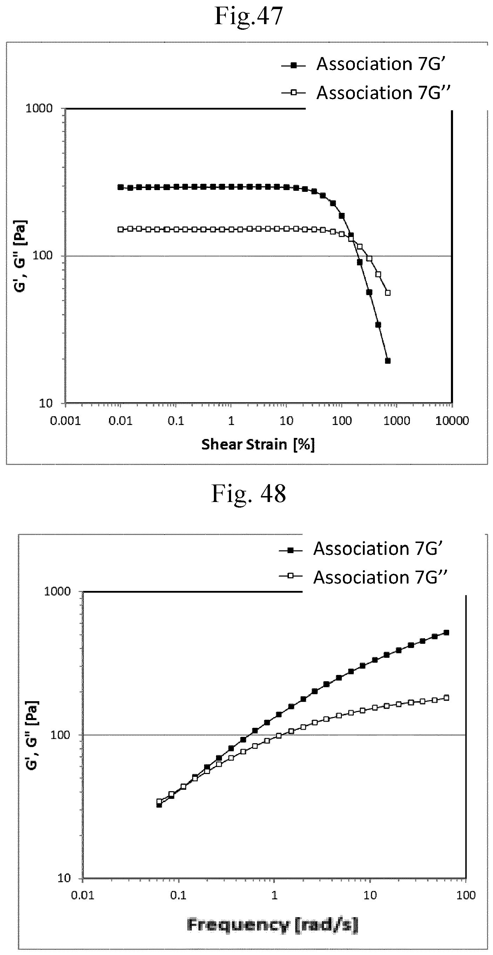

The predominantly viscous contribution of Hydroxypropyl guar gum, Cellulose gum, algin and low molecular weight Sodium hyaluronate on the weak gel structure of Sclerotium gum gives rise to a structure with a predominantly elastic character. The elastic module G' remains higher than the viscous module G" throughout the frequency range but the module varies strongly with the frequency (fig 48), thus placing Association 7 between the viscoelastic materials and the weak gels.

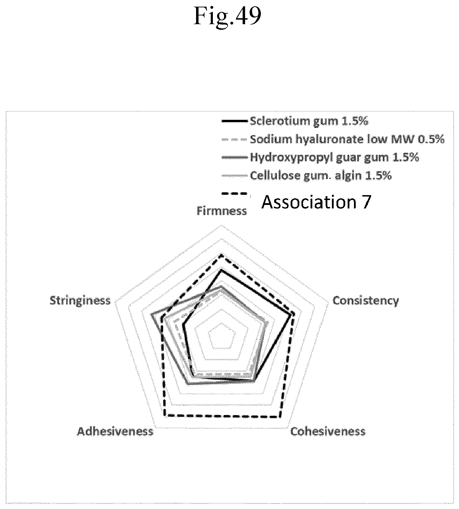

Comparing the texture properties, very high adhesivity and cohesiveness values are observed for association 7 which the individual polymers are not able to reach (figure 49). Thus, a product with features suitable for application on the oral mucosa is obtained, features which could not be satisfied with the use of a single polymer.

The parameters of association 7 fall within those identified for a product suitable for application on oral mucosa:
G^{∗} (at 1Hz): 332 Pa
Tan δ (at 1Hz): 0.5
Adhesivity (A-): 2.31 N.mm

### Association 8:

**Polymers used (4 polymers including 1 weak gel, 2 viscoelastic gels, 1 viscous gel):**

| Polysaccharide (class) | % used in the association: | |
|---|---|---|
| Modified potato starch | 4% | |
| Hydroxypropyl guar gum | 1% | 20 |
| Pectin | 0.5% | |
| Sodium hyaluronate high MW | 0.1% | |

The rheological analyses under oscillatory conditions of the polymers forming Association 8 are shown below.

### RHEOLOGICAL CHARACTERIZATION OF Modified potato starch

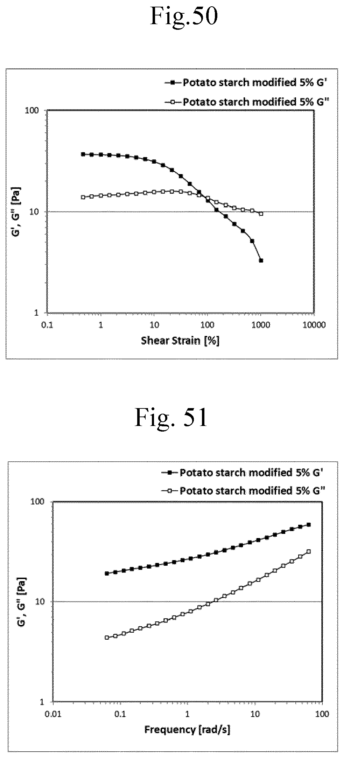

The Modified potato starch-based gel has rheological behaviour, obtained as a function of the oscillation frequency at constant strain amplitude (frequency sweep) typical of a weak gel, i.e., it has the following features:
- the elastic component G' is always higher than the viscous component G" throughout the frequency range investigated;
- the absolute values of the two modules are comprised between the second and the third set of ten (medium-low quantitative rheological response). (figure 51).

### RHEOLOGICAL CHARACTERIZATION OF Hydroxypropyl guar gum

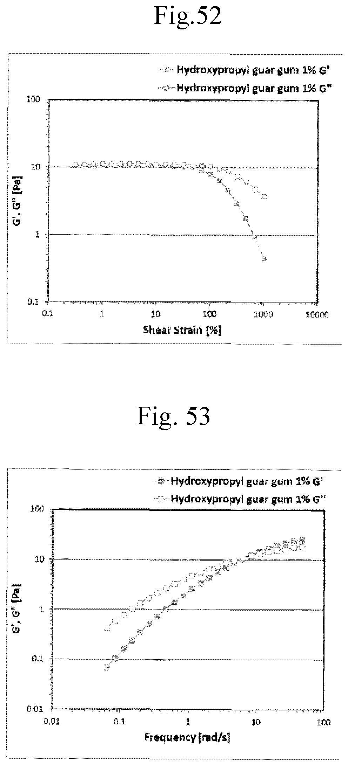

The Hydroxypropyl Guar gum-based sample has viscoelastic behaviour, in which the elasticity at high frequencies prevails (the elastic module G' is greater than the viscous module G"), while at low frequencies the situation is reversed with the prevalence of the viscous character (the module G" greater than the module G') (figure 53). The crossing point between the two modules is located at a frequency of 8.38 rad/s.

### RHEOLOGICAL CHARACTERIZATION OF Pectin

The polymer forms a structured solution of predominantly viscous character.

G", viscous module, is always higher than the elastic component G' both in As and as a function of the frequency. Both modules are sensitive to frequency variations (trends decrease as the applied frequency decreases) and the quantitative responses are low.

### RHEOLOGICAL CHARACTERIZATION OF Sodium hyaluronate high MW

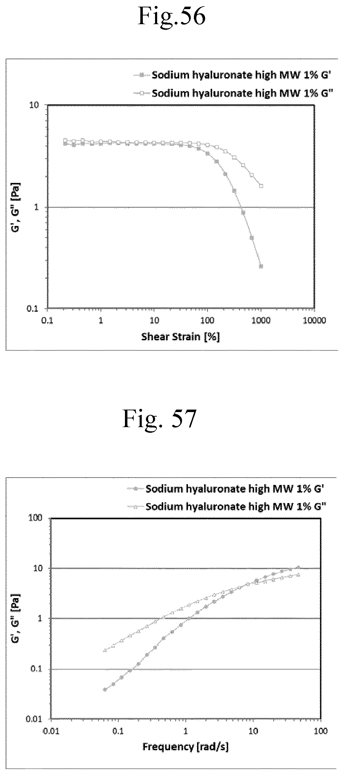

The Sodium hyaluronate high MW-based sample has viscoelastic behaviour, in which the elasticity at high frequencies prevails (the elastic module G' is greater than the viscous module G"), while at low frequencies the situation is reversed with the prevalence of the viscous character (the module G" greater than the module G') (figure 57).

### RHEOLOGICAL CHARACTERIZATION AND TEXTURE OF ASSOCIATION 8: 4 polymers including 1 weak gel, 1 viscoelastic gel, 2 viscous gels

Modified potato starch 4%, Hydroxypropyl guar gum 1%, Pectin 0.5%, Sodium hyaluronate high MW 0.1%

Similar to Association 7, the contribution of polymers with a predominantly viscous/viscoelastic character (in this case Hydroxypropyl guar gum, Pectin, Sodium hyaluronate high MW) on the structure of the polymer weak gel (Modified potato starch) gives rise to a structure with a predominantly elastic character with peculiar features: the elastic module G' remains higher than the viscous module G" throughout the frequency range but the modules vary strongly with the frequency (fig 57). The Association 8 is placed between the viscoelastic materials and the weak gels showing a different rheological trend with respect to the individual polymers which compose it.

The texture features of Association 8 are similar in firmness and consistency to those of the modified potato starch gel but have greater adhesivity and cohesiveness with respect to those recorded in the single polymer gels. It emerges once again how, by combining different polymers, gels can be obtained with adhesivity which cannot be reached with a single polymer.

**The parameters of association 8 fall within those identified for a product suitable for application on oral mucosa:**
**G* (at 1Hz): 311 Pa**
**Tan δ (at 1Hz): 0.4**
**Adhesivity (A-): 1.62 N.mm**

## Claims

1. Mucosal composition containing a mixture comprising at least three polysaccharides belonging to at least two of the following classes 1, 2, 3 and 4, as classified in Table 1 below
| | | Class 1, ELASTIC gels | | Class 2, WEAK gels | | Class 3, VISCOELASTIC gels | Class 4, VISCOUS gels |
|---|---|---|---|---|---|---|---|
| Tan δ | <<1 | | <1 | | >1 at low frequencies (e.g., 0.1 Hz), <1 at high frequencies (e.g., 10Hz) | | >1 |
| Adhesivity (A-) | 0.05 - 0.06 N.mm | | 0.1-0.5 N.mm | | 0.5-2.5 N.mm | | 0.01-0.5 N.mm |
in combination with suitable excipients and/or diluents,
in which said mixture has:
a) at 1Hz: complex module G^{∗} comprised between 1 and 500 Pa and Tan δ comprised between 0.3 and 1
b) Adhesivity A- 0.08 - 5 N.mm, and
c) the relative polysaccharides chosen in Table 2 below
**TABLE 2**
| **INCI** | **REOLOGICAL FEATURES (class)** | **RANGE %** |
|---|---|---|
| Gellan Gum (high acylation) | ELASTIC GELS (1) | 0.1 - 0.5 |
| Iota Carrageenan (Chondrus crispus) | ELASTIC GELS (1) | 0.20 - 0.75 |
| Xanthan Gum | WEAK GELS (2) | 0.5 - 1.5 |
| Sclerotium Gum | WEAK GELS (2) | 1.0 -2.0 |
| *Citrus limon peel powder, Sclerotium gum* | WEAK GELS (2) | 1.0 -2.0 |
| Succinoglycan | WEAK GELS (2) | 0.2- 1.0 |
| Acacia Senegal Gum | WEAK GELS (2) | 0.75 - 1.5 |
| Potato starch modified | WEAK GELS (2) | 3.0 -5.0 |
| Astragalus Gummifer Gum | WEAK GELS (2) | 1.0 - 2.0 |
| Hydroxypropyl starch phosphate | WEAK GELS (2) | 5.0 - 6.0 |
| Amorphophallus Konjac Root Extract | VISCOELASTIC GELS (3) | 0.75 - 1.25 |
| Hydroxypropyl Guar | VISCOELASTIC GELS (3) | 1.0 - 2.5 |
| Caesalpinia spinosa gum | VISCOELASTIC GELS (3) | 1.0 - 2.0 |
| Ceratonia siliqua (carob) gum | VISCOELASTIC GELS (3) | 0.5 - 2.0 |
| Sodium hyaluronate medium-high MW | VISCOELASTIC GELS (3) | 0.01 -2.0 |
| Tamarindus indica seed polysaccharide medium-high MW | VISCOELASTIC GELS (3) | 0.01 -2.0 |
| C 18-22 Hydroxyalkyl hydroxypropyl guar | VISCOELASTIC GELS (3) | 0.5 - 1.5 |
| *Cellulose gum, Algin* | VISCOELASTIC GELS (3) | 1.0 - 2.0 |
| Pectin | VISCOUS GELS (4) | 0.5 - 6.0 |
| Tamarindus indica seed polysaccharide (low MW) | VISCOUS GELS (4) | 0.1 - 3.0 |
| Sodium hyaluronate (low MW) | VISCOUS GELS (4) | 0.5 - 2.0 |

2. Mucosal composition according to claim 1, wherein:
a) at 1Hz: G^{∗} is comprised between 150 and 500 Pa and tan δ is comprised between 0.3 and 1
b) adhesivity A- is comprised between 0.8 and 5 N.mm,
and can be administered in the oral cavity.

3. Mucosal composition according to claim 1, wherein:
a) at 1Hz: G^{∗} is comprised between 1 and 150 Pa and tan δ is comprised between 0.4 and 1
b) adhesivity is comprised between 0.08 -1.2 N.mm,
and is administered in the pharyngeal nose tract.

4. Oral mucosal composition according to claim 2, comprising between 1 and 2%, preferably 1% of Sclerotium gum, between 1 and 2%, preferably 1% of Caesalpinia spinosa gum, and between 0.5 and 2%, preferably 0.5% of Ceratonia siliqua (carob) gum by weight of the total weight of the mucosal composition.

5. Nasally administrable mucosal composition according to claim 3, comprising between 0.2 and 1%, preferably 0.2% of Succinoglycan, between 0.2 and 0.75%, preferably 0.2% of Carrageenan (iota carrageenan), between 0.1 and 3%, preferably 0.2% of Tamarindus Indica Seed Polysaccharide.

6. Nasally administrable mucosal composition of claim 5, comprising pharmaceutically acceptable alkaline earth metal salts, preferably calcium chloride and magnesium sulphate.

7. Oral mucosal composition of claim 2, comprising: between 1 and 2%, preferably 1.25% of Sclerotium gum, between 0.5 and 2%, preferably 1% of Ceratonia siliqua (carob) gum and between 0.5-6%, preferably 2% of pectin.

8. Oral mucosal composition according to claim 2, comprising between 1 and 2%, preferably 1.25% of Sclerotium gum; between 0.5 and 2%, preferably 1% of Ceratonia siliqua (carob) gum, between 0.5 and 6% by weight, preferably 0.5% of pectin and between 0.1 and 3%, preferably 0.5% of Tamarindus indica seed polysaccharide.

9. Oral mucosal composition according to claim 2, comprising between 1 and 2%, preferably 1 % of Sclerotium gum; between 0.5 and 2%, preferably 0.5% of Ceratonia siliqua (carob) gum; between 0.5 and 2%, preferably 1% of Caesalpinia spinosa gum; and between 0.1 and 3%, preferably 0.1% of Tamarindus indica seed polysaccharide.

10. Oral mucosal composition according to claim 2, containing Hydroxy propyl guar gum between 1.0 and 2.5%, preferably 1% by weight on the total weight of the mucosal composition, Sclerotium 10 gum in amounts comprised between 1 and 2% by weight, preferably 1% by weight on the total weight of the mucosal composition; Cellulose gum algin in compositions between 1 and 2% by weight, preferably 1% by weight on the total weight of the composition, Sodium hyaluronate (Low MW) in concentration comprised between 0.5 and 2%, preferably 0.5% by weight on the total weight of said composition.

11. Oral mucosal composition according to claim 2, containing Potato starch modified at concentrations comprised between 3 and 5%, preferably 4% by weight of the total weight of the mucosal composition; Hydroxypropyl guar gum at concentrations comprised between 1 and 2.5%, preferably 1% by weight of the total weight of the mucosal composition, pectin at concentrations comprised between 0.5 and 6% by weight, preferably 0.5%, Sodium hyaluronate High MW at concentrations comprised between 0.01 and 2%, preferably 0.1% by weight of the total weight of the mucosal composition.

12. Mucosal composition according to any one of claims 1-11, for use in the treatment and prevention of viral and bacterial infections.
